(19) Europäisches Patentamt
European Patent Office
Office européen des brevets

(11) Veröffentlichungsnummer : **0 332 805 B1**

# (12) EUROPÄISCHE PATENTSCHRIFT

(45) Veröffentlichungstag der Patentschrift : **04.03.92 Patentblatt 92/10**

(51) Int. Cl.$^5$ : **A61K 7/075,** C11D 17/00, A61K 7/50

(21) Anmeldenummer : **89100697.5**

(22) Anmeldetag : **17.01.89**

## (54) Fliessfähige Perlglanzdispersion und deren Verwendung.

(30) Priorität : **17.03.88 DE 3808881**

(43) Veröffentlichungstag der Anmeldung : **20.09.89 Patentblatt 89/38**

(45) Bekanntmachung des Hinweises auf die Patenterteilung : **04.03.92 Patentblatt 92/10**

(84) Benannte Vertragsstaaten : **AT BE CH DE ES FR GB IT LI NL SE**

(56) Entgegenhaltungen :
**EP-A- 0 195 251**
**EP-A- 0 268 992**
**DE-A- 3 519 080**

(73) Patentinhaber : **HÜLS AKTIENGESELLSCHAFT**
**Patentabteilung / PB 15 - Postfach 13 20**
**W-4370 Marl 1 (DE)**

(72) Erfinder : **Ruback, Wulf, Dr.**
**Moltkestrasse 15 a**
**W-4350 Recklinghausen (DE)**
Erfinder : **Pohl, Heinz**
**Dürerstrasse 43**
**W-4370 Marl (DE)**

EP 0 332 805 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Zur Verbesserung des optischen Eindruckes und damit zur Steigerung des Handelswertes werden wäßrige Zubereitungen von Tensiden, wie kosmetische Haar- und Körperreinigungsmittel oder auch flüssige Wasch- und Reinigungsmittel, oft mit einem perlglänzenden Aussehen ausgestattet. Um einen solchen Perl- oder auch Seidenglanzeffekt zu erreichen, wurden früher pulverförmige Naturstoffe wie Glimmer oder Fischsilber, anorganische Materialien wie Titandioxidpigmente oder auch Metallsalze höherer Fettsäuren eingesetzt.

Seit einiger Zeit werden für diesen Zweck Fettsäureglykolester allein oder in Kombination mit Fettsäurealkanolamid verwendet. (G. A. Nowak "Die kosmetischen Präparate" Band 2, Seite 482, Augsburg 1984). Diese beiden Materialien sind feste, nichtkristalline Substanzen. Zur Herstellung von wäßrigen Tensidzubereitungen mit Perlglanzeffekt muß der Ansatz über den Schmelzpunkt dieser Substanzen erwärmt werden. Hierbei wird eine homogene Lösung erhalten, aus der nach dem Abkühlen durch Kristallisation ein Perl- bzw. Seidenglanzeffekt entsteht. Dieses Verfahren besitzt jedoch den Nachteil, daß hierbei der gesamte Produktionsansatz auf eine entsprechende Temperatur (je nach Perlglanzbildner ca. 60 bis 80 °C) erhitzt werden muß und erst durch definierte Abkühl- und Rührbedingungen ein gleichmäßiger Perlglanz entsteht. Dies bedingt einen hohen Energie- und Zeitaufwand.

Hierzu kommt, daß bei diesen Zubereitungen wärmeempfindliche oder flüchtige Formulierungsbestandteile, wie Parfümöle oder spezielle Wirkstoffe, erst nach dem Abkühlen zudosiert werden können. Dadurch gibt es keine Möglichkeit der kontinuierlichen Produktion solcher perlglänzender Formulierungen.

In jüngerer Zeit werden nun sogenannte Perlglanzdispersionen, das sind Kombinationen der verschiedensten Perlglanzbildner mit Tensiden, eingesetzt, mit denen es möglich ist, flüssige Formulierungen mit Perlglanzeffekt ohne Erhitzen und auch kontinuierlich zu produzieren. Diese Perlglanzdispersionen werden hergestellt, indem die perlglanzgebende Substanz oberhalb ihrer Schmelztemperatur in die wäßrige Lösung des Tensids eingearbeitet wird. Durch konstante, exakt abgestimmte Abkühl- und Rührbedingungen werden hierbei Perlglanzkristalle mit einer definierten Teilchengrößenverteilung gebildet, die in der Tensidlösung dispergiert bleiben.

Die derzeit am häufigsten verwendeten Substanzen zur Herstellung dieser Perlglanzdispersionen sind Fettsäuremono- und/oder -polyglykolester, die allein oder zusammen mit Fettsäurealkanolamiden in wäßrige Lösungen von Alkylsulfaten und/oder Alkylethersulfaten als Dispergiermittel eingearbeitet werden. In den meisten Fällen werden hierbei sehr hochviskose, bei tieferen Temperaturen nicht mehr fließfähige Dispersionen erhalten. (DE-OS 16 69 152, DE-OS 35 19 080)

Nun ist es bei Perlglanzdispersionen dieser Art erforderlich, einen recht hohen Anteil der anionischen Tenside vom Sulfat-Typ zu verwenden. Da aber die Nachfrage nach milden Präparaten immer größer wird, möchte man sich nicht solche "formulierungsfremden" Tenside durch einen Hilfsstoff, wie z. B. das Perlglanzmittel, einschleppen.

In DE-A 34 21 161 scheint diese Aufgabe in gewisser Weise gelöst, indem eine bekannte Mischung von Fettsäureglykolester und Fettsäuremonoalkylolamid mit Hilfe einer speziellen Kombination von Sulfosuccinat und Aminoxid zu einem Perlglanzmittel verarbeitet wird. Perlglanzkonzentrate, die nach diesem Verfahren hergestellt werden (vgl. die dort aufgeführten Beispiele) haben jedoch eine für die Weiterverarbeitung unerwünscht hohe Viskosität, und der mit ihnen erzielbare Perlglanzeffekt entspricht nicht ganz dem gewohnten Standard. Ferner sind bei der Herstellung bestimmte Dosierungsreihenfolgen und Temperaturprofile einzuhalten, d. h. die Herstellungsverfahren sind aufwendig.

Es bestand daher die Aufgabe, Perlglanzkonzentrate mit einem ausgezeichneten Perlglanzeffekt aufzufinden, die einerseits bei Raumtemperatur gut fließfähig, andererseits aber auch über lange Zeit lagerstabil sind, dabei einen möglichst geringen Anteil an milden Tensiden bzw. Dispergiermitteln enthalten und sich einfach herstellen lassen.

Überraschend wurde gefunden, daß durch die Kombination von Fettsäuremono- oder -di(poly)glykolestern und Fettsäurealkylolamiden mit geringen Mengen einer Mischung aus ggf. oxethylierten Fettaminen und Carbonsäuren die geforderten Kriterien ausgezeichnet erfüllt werden. Die erfindungsgemäße, fließfähige Perlglanzdispersion besteht aus folgenden Komponenten:

A) 5 bis 25, vorzugsweise 10 bis 20, Gew.-% eines Fettsäureglykolesters der allgemeinen Formel I

$$R_1-\overset{O}{\overset{\|}{C}}-(O-A)_n-O-X \qquad (I)$$

darin steht

$R_1$ für eine lineare oder verzweigte, gesättigte oder ungesättigte Kohlenwasserstoffkette mit 14 bis 23 Kohlenstoffatomen,

A für eine Gruppe der Formel $-C_2H_4-$ oder $C_3H_6-$, vorzugsweise $-C_2H_4-$,

X für ein Wasserstoffatom oder eine Gruppe der allgemeinen Formel

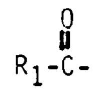

n für eine Zahl von 1 bis 5, vorzugsweise 1 bis 2,

B) 2 bis 15, vorzugsweise 2 bis 10, Gew.-% eines Fettsäurealkanolamides der allgemeinen Formel II

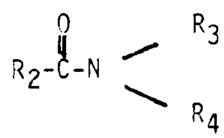

(II)

darin steht,

$R_2$ für eine lineare oder verzweigte, gesättigte oder ungesättigte Kohlenwasserstoffkette mit 9 bis 23 Kohlenstoffatomen,

$R_3$ und $R_4$ unabhängig voneinander für ein Wasserstoffatom oder eine Gruppe der Formel $-C_2H_4OH$ oder $-C_3H_6OH$,

C) 0,1 bis 5 Gew.-%, vorzugsweise 0,3 bis 3 Gew.-%, eines Amins der allgemeinen Formel III

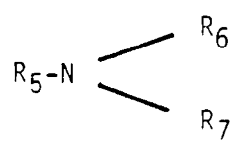

(III)

darin steht

$R_5$ für eine lineare oder verzweigte, gesättigte oder ungesättigte Kohlenwasserstoffgruppe mit 8 bis 24 Kohlenstoffatomen,

$R_6$ für ein Wasserstoffatom der eine lineare oder verzweigte Kohlenstoffkette mit 1 bis 4 Kohlenstoffatomen, oder eine Gruppe der Formel $-(A-O)_m-H$, worin A die o. a. Bedeutung hat,

$R_7$ für eine Gruppe der Formel $-(A-O)_oH$ oder einen Polyhydroxyalkylrest mit 3 bis 7, vorzugsweise 4 bis 6 Kohlenstoffatomen, der ggf. mit einem Mono-, Di- oder Oligosaccharid glykosidisch verknüpft ist; dabei sind

m und o jeweils unabhängig voneinander ganze Zahlen von 0 bis 40, bevorzugt 1 bis 30, wobei die Summe von m plus o eine ganze Zahl von 1 bis 50 ist.

D) 0,1 bis 5 Gew.-%, vorzugsweise 0,3 bis 3 Gew.-%, einer Carbonsäure der allgemeinen Formel IV

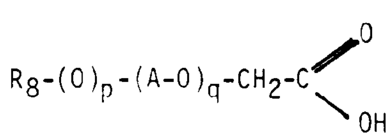

(IV)

darin steht

$R_8$ für eine lineare oder verzweigte, gesättigte oder ungesättigte Kohlenwasserstoffgruppe mit 7 bis 22, vorzugsweise 8 bis 20, Kohlenstoffatomen,

A für die gleiche Bedeutung wie in Formel I,

p für 0 oder 1 und

q für eine ganze Zahl zwischen 0 und 16, vorzugsweise 0 bis 10, wobei p gleich 1 ist, wenn q größer als 0 ist,

E) 0 bis 5 Gew.-%, vorzugsweise 0,1 bis 4 Gew.-%, insbesondere 0,3 bis 2,5 Gew.-% eines Sulfonates der allgemeinen Formel V

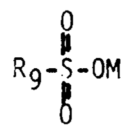

(V)

oder eines Sulfates der allgemeinen Formel VI

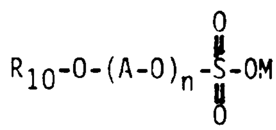

(VI)

oder eines Gemisches davon
darin steht

$R_9$ für einen Phenylrest mit 1 bis 3 Substituenten, vorzugsweise aus der Gruppe der Kohlenwasserstoffreste, wobei die Summe der Kohlenstoffatome dieser Substituenten eine Zahl von 1 bis 9, vorzugsweise von 1 bis 6, insbesondere von 1 bis 3, ist,

$R_{10}$ für einen linearen oder verzweigten Kohlenwasserstoffrest mit 1 bis 10, vorzugsweise 2 bis 6, Kohlenstoffatomen,

M für ein Atomäquivalent eines ein- oder zweiwertigen Metalls, vorzugsweise eines Alkalimetalls, oder eine Ammonium- oder substituierte Ammoniumgruppe,

während A und n die in Formel I angegebene Bedeutung haben,

F) 0 bis 2 Gew.-%, vorzugsweise 0 bis 1 Gew.-%, eines ein- oder zweiwertigen Metallsalzes, vorzugsweise Alkalisulfat oder Alkalichlorid, sowie

Wasser zu der an 100 Gew.-% fehlenden Menge.

Die erfindungsgemäße Perlglanzdispersion sollte 5 bis 40, vorzugsweise 10 bis 30 insbesondere 15 bis 25 Gew.-% der Komponenten A bis F bzw. A bis D enthalten. Das Mengenverhältnis A zu B sollte 6 : 1 bis 1 : 1 betragen.

In einer bevorzugten Ausführungsform der vorliegenden Erfindung werden vorzugsweise 10 bis 20 Gew.-%, insbesondere 14 bis 18 Gew.-% der Komponente A eingesetzt, wobei die Eigenschaften der erfindungsgemäßen Perlglanzdispersion am günstigsten sind, wenn $R_1$ ein Alkylrest mit 15 bis 17 Kohlenstoffatomen, X ein Rest der Formel

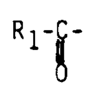

und n = 1 ist.

Als Komponente B kommen bevorzugt Verbindungen zum Einsatz, in denen gemäß Formel II $R_2$ eine Kohlenwasserstoffkette mit 11 bis 19 Kohlenstoffatomen, $R_3$ eine Gruppe der Formel $-C_2H_4OH$ und $R_4$ ein Wasserstoffatom darstellen. Komponente B wird vorzugsweise in einer Konzentration von 2 bis 10 Gew.-%, insbesondere von 3 bis 5 Gew.-%, eingesetzt.

Die bevorzugten Einsatzkonzentrationen der Komponenten C und D liegen bei jeweils 0,3 bis 3 Gew.-%, insbesondere 0,5 bis 2 Gew.-%. Als Amin gemäß der allgemeinen Formel III werden erfindungsgemäß bevorzugt Verbindungen eingesetzt, in denen $R_5$ für eine gesättigte oder ungesättigte Kohlenwasserstoffgruppe mit 12 bis 20, insbesondere 12 bis 18, Kohlenstoffatomen, $R_6$ und $R_7$ gleichzeitig eine Gruppe der Formel $-(A-O)_wH$, insbesondere der Formel $-(C_2H_4O)_wH$, ist, worin w jeweils m bzw. o bedeutet, wobei die Summe von m und o insbesondere eine Zahl von 4 bis 25 ist.

Als Carbonsäure werden in einer bevorzugten Ausführungsform der Erfindung Verbindungen gemäß der allgemeinen Formel IV eingesetzt, in denen $R_5$ für einen gesättigten oder ungesättigten Kohlenwasserstoffrest mit 8 bis 20 Kohlenstoffatomen steht, A die Bedeutung $-C_2H_4-$ hat und q eine Zahl von 0 bis 10 ist. In einer besonders bevorzugten Ausführungsform ist $R_8$ ein Kohlenwasserstoffrest mit 14 bis 18 Kohlenstoffatomen,

und p und q haben den Wert 0.

Beispiele für Verbindungen, die als Komponente A) infrage kommen, sind:

Palmitinsäure-, Stearinsäure-, Arachinsäure-, Behensäure-monoethylenglykolester oder -monoisopropylenglykolester; statt der Monoglykolester sind auch die Di-, Tri-, Tetra- oder Pentaethylenglykolester bzw. Pentapropylenglykolester möglich. Auch Monoethylenglykol- dipalmitat, -distearat, -diapachinat, -dibehenat, -dilignocerinat, -dioleat, können eingesetzt werden. In den genannten Verbindungen kann das Monoethylenglykol auch durch Diethylenglykol, Triethylenglykol, Tetraethylenglykol oder Pentaethylenglykol substituiert werden. Ebenso kann darin Ethylenglykol durch Isopropylglykol ersetzt sein.

Beispiele für Verbindungen, die als Komponente B) herangezogen werden können, sind:

Mono- oder Diethanolamide oder auch Mono- oder Diisopropanolamide der Caprinsäure, Laurinsäure, Myristinsäure, Palmitinsäure, Stearinsäure, Arachinsäure, Behensäure, Lignocerinsäure, Ölsäure oder der Mischungen von Fettsäuren wie Kokosnuß- oder Palmkernfettsäuren.

Als Komponente C) lassen sich beispielsweise einsetzen:

Capryl-, Caprinyl-, Lauryl-, Oleyl-, Myristyl-, Palmityl-, Stearyl-, Behenyl-, Talg-, Arachinyl-, Lignoarylamin, jeweils umgesetzt mit 1 bis 50, vorzugsweise 1 bis 30 Molen, Ethylenoxid oder 1,2-Propylenoxid.

Als Komponente D) kommen infrage:

Caprin, Capryl-, Laurin-, Myristin-, Palmitin-, Palmitolein-, Stearin-, Öl-, Arachidon,- Behensäure sowie Capryl-, Caprinyl-, Lauryl-, Myristyl-, Palmityl-, Stearyl-, Arachinyl- Behenyl-, Oleyl-Carboxymethylat, wobei an die den Alkylresten zugrundeliegenden Alkohole vor der Carboxymethylierung 1 bis 16 Mole Ethylenoxid oder 1,2-Propylenoxid angelagert werden kann. Die Carboxymethylierung kann in bekannter Weise durch Umsatz der Alkohole bzw. der Alkyl(poly)oxalkylate mit Chloressigsäure und NaOH erfolgen.

Als Komponente E) lassen sich einsetzen:

Toluolsulfonat, Xylolsulfonat, Cumolsulfonat, Ethylbenzolsulfonat oder:

Methyl-, Ethyl-, Propyl-, Butyl-, Pentyl-, Hexyl-, Octyl-, Decylsulfat, wobei die den Alkylresten zugrundeliegenden Alkohole vor der Sulfatierung mit 1 bis 5 Molen Ethylenoxid oder 1,2-Propylenoxid umgesetzt werden können.

Zur Herstellung der erfindungsgemäßen Perlglanzdispersionen wird zweckmäßigerweise die entsprechende Menge Wasser von 70 bis 80 °C, bevorzugt 75 °C, vorgelegt und die Komponenten A bis E werden unter nicht zu heftigem Rühren (< 100 UpM) in beliebiger Reihenfolge zugegeben. Währenddessen wird die zum Schmelzen und Dispergieren der Komponenten benötigte Energie zugeführt, so daß die Temperatur im Kessel im angegebenen Bereich bleibt. Anschließend kann ggf. Komponente F zugesetzt werden. Nach einer Nachrührzeit von 0,5 bis 1 Stunde wird man unter langsamen Rühren mit ca. 5 °C/Stunde auf mindestens 50 °C, bevorzugt 40 °C, abkühlen lassen, wobei sich der gewünschte seidige Perlglanz einstellt. Um eine mikrobielle Kontamination zu vermeiden, sollte man der Perlglanzdispersion ein geeignetes Konservierungsmittel zusetzen.

Die solchermaßen hergestellten erfindungsgemäßen Perlglanzdispersionen zeichnen sich durch die folgenden Vorteile aus:

1. Sie sind sehr einfach in einem Eintopfverfahren herstellbar und unkritisch in bezug auf das Kristallisationsverhalten beim Abkühlen.
2. Sie sind - auch unterhalb Raumtemperatur - sehr gut fließfähig, wodurch sie für eine kontinuierliche Weiterverarbeitung prädestiniert sind.
3. Sie haben einen sehr niedrigen Anteil an nicht perlglanzgebenden Komponenten, wodurch sich erheblich vielfältigere Einsatzmöglichkeiten ergeben.
4. Nicht nur die Fließfähigkeit der erfindungsgemäßen Perlglanzdispersion ist verbessert, sondern gleichzeitig erhält man einen einheitlicheren seidigeren Perlglanzeffekt.

Die vorliegende, erfindungsgemäße Perlglanzdispersion kann bei Raumtemperatur flüssigen Haar- und Körperreinigungsmitteln, flüssigen Geschirrspülmitteln sowie flüssigen Wasch- und Reinigungsmitteln zugesetzt werden. Hierdurch werden Fertigprodukte erhalten, die einen sehr feinen und intensiven Perlglanz aufweisen. Die hierzu benötigte Menge der Perlglanzdispersion beträgt 0,5 bis 6 %, vorzugsweise 1 bis 4 %, insbesondere 1,5 bis 3 Gew.-%.

Die folgenden Beispiele dienen zur näheren Erläuterung der erfindungsgemäßen Perlglanzdispersion. Die Mengenangaben beziehen sich jeweils auf Gewichtsprozente. Die Herstellung erfolgte in allen Fällen auf die oben beschriebene Art und Weise.

Zusammensetzung in Gew.-%

| | Beispiel-Nr. | | | | | | | | |
|---|---|---|---|---|---|---|---|---|---|
| Komponenten | 1 | 2 | 3 | 4 | 5 | 6 | 7 | 8 | 9 |
| Monoethylenglykol-distearat | 16 | 16 | 10 | 16 | 16 | 16 | 10 | 16 | 16 |
| Cocosfettsäuremono-ethanolamid | 4 | 4 | 10 | 4 | 4 | 4 | 10 | 4 | 4 |
| Laurylamin-10 EO | 1 | | 1 | | 1 | | | | |
| Talgamin-10 EO | | 1 | | | | | | 1 | |
| Oleylamin-10 EO | | | | | | | | | 1 |
| Oleylamin-8 EO | | | | 1 | | | | | |
| N-Dodecylglucamin | | | | | | 1 | 1 | | |
| Ölsäure | 1 | 1 | 1 | 1 | | | | | 1 |
| $C_{1214}$-4.5EO-essigsäure*) | | | | | | 1 | | 1 | |
| $C_{1214}$-10EO-essigsäure*) | | | | | 1 | | 1 | | |
| Cumolsulfonat | 1 | 1 | | 1 | | | | 1 | 1 |
| Natriumsulfat | | | 1 | | 1 | 1 | 1 | | |

*) Formel IV: $R_8$ = Lauryl und Myristyl, q = 4,5 bzw. 10

Alle in den Beispielen aufgeführten Zusammensetzungen sind gut fließfähig und geben bei Zusatz zu verschiedenen Tensid-Zubereitungen einen hervorragenden Perlglanz, wobei dieser bei den erfindungsgemäßen Beispielen 1, 2 und 4 besonders fein erscheint.

## Patentansprüche

1. Fließfähige Perlglanzdispersionen, bestehend aus

A) 5 bis 25 Gew.-% eines Fettsäureglykolesters der allgemeinen Formel I

$$R_1-\overset{\overset{O}{\|}}{C}-(O-A)_n-O-X \qquad (I),$$

in der

$R_1$ für eine lineare oder verzweigte, gesättigte oder ungesättigte Kohlenwasserstoffkette mit 14 bis 23 Kohlenstoffatomen,

A für eine Gruppe der Formel $-C_2H_4-$ oder

$$-\underset{\underset{CH_3}{|}}{CH}-CH_2-,$$

X für ein Wasserstoffatom oder eine Gruppe der allgemeinen Formel

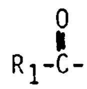

n für eine Zahl von 1 bis 5, vorzugsweise 1 bis 2, steht,

B) 2 bis 15 Gew.-% eines Fettsäurealkanolamides der allgemeinen Formel II

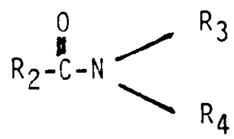 (II),

in der

$R_2$ eine lineare oder verzweigte, gesättigte oder ungesättigte Kohlenwasserstoffkette mit 9 bis 23 Kohlenstoffatomen,

$R_3$ und $R_4$ unabhängig voneinander ein Wasserstoffatom oder eine Gruppe der Formel $-C_2H_4OH$ oder

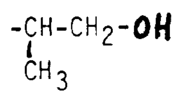

bedeuten,

C) 0,1 bis 5 Gew.-% eines Amins der allgemeinen Formel III

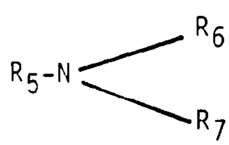 (III),

in der

$R_5$ für eine lineare oder verzweigte, gesättigte oder ungesättigte Kohlenwasserstoffgruppe mit 8 bis 24 Kohlenstoffatomen,

$R_6$ für ein Wasserstoffatom oder eine lineare oder verzweigte Kohlenstoffkette mit 1 bis 4 Kohlenstoffatomen, oder eine Gruppe der Formel $-(A-O)_m-H$, worin A die o. a. Bedeutung hat,

$R_7$ für eine Gruppe der Formel $-(A-O)_oH$ oder einen Polyhydroxyalkylrest mit 3 bis 7, vorzugsweise 4 bis 6, Kohlenstoffatomen steht, der ggf. mit einem Mono-, Di- oder Oligosaccharid glykosidisch verknüpft ist, wobei

m und o jeweils unabhängig voneinander ganze Zahlen von Null bis 40, bevorzugt 1 bis 30, bedeuten, deren Summe m plus o eine ganze Zahl von 1 bis 50 sein kann,

D) 0,1 bis 5 Gew.-% einer Carbonsäure der allgemeinen Formel IV

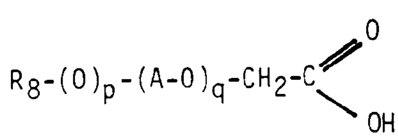 (IV),

in der

$R_8$ für eine lineare oder verzweigte, gesättigte oder ungesättigte Kohlenwasserstoffgruppe mit 7 bis 22, vorzugsweise 8 bis 20, Kohlenstoffatomen steht,

A die gleiche Bedeutung hat, wie in Formel I,

p 0 oder 1 ist und

q für eine ganze Zahl zwischen 0 und 16 steht, wobei p gleich 1 ist, wenn q größer als 0 ist,

E) 0 bis 5 Gew.-%, vorzugsweise 0,1 bis 4 Gew.-%, eines Sulfonates der allgemeinen Formel V

$$R_9-\underset{\underset{O}{\|}}{\overset{\overset{O}{\|}}{S}}-OM \qquad (V)$$

oder eines Sulfates der allgemeinen Formel VI

$$R_{10}-O-(A-O)_n-\underset{\underset{O}{\|}}{\overset{\overset{O}{\|}}{S}}-OM \qquad (VI)$$

oder eines Gemisches davon, worin

$R_9$ einen Phenylrest mit 1 bis 3 Substituenten, vorzugsweise aus der Gruppe der Kohlenwasserstoffreste, bedeutet, wobei die Summe der Kohlenstoffatome dieser Substituenten eine Zahl von 1 bis 9, vorzugsweise von 1 bis 6, sein kann,

$R_{10}$ für einen linearen oder verzweigten Kohlenwasserstoffrest mit 1 bis 10, vorzugsweise 2 bis 6, Kohlenstoffatomen,

M für ein Atomäquivalent eines ein- oder zweiwertigen Metalls, vorzugsweise eines Alkalimetalls, oder eine Ammonium- oder substituierte Ammoniumgruppe steht, während

A und n die in Formel I angegebenen Bedeutungen haben,

F) 0 bis 2 Gew.-%, vorzugsweise 0 bis 1 Gew.-%, eines ein- oder zweiwertigen Metallsalzes, vorzugsweise Alkalisulfat oder 5 Alkalichlorid, sowie

G) Wasser zu der an 100 Gew.-% fehlenden Menge.

2. Verwendung der fließfähigen Perlglanzdispersion nach Anspruch 1 als Zusatz zu wäßrigen Tensid-Zubereitungen.

## Claims

1. A free-flowing dispersion of pearly lustre composed of

A) from 5 to 25% by weight of a fatty acid glycol ester of the general formula I

$$R_1-\overset{\overset{O}{\|}}{C}-(O-A)_n-O-X \qquad (I)$$

where

$R_1$ is a linear or branched, saturated or unsaturated hydrocarbon chain of from 14 to 23 carbon atoms,

A is a group of the formula $-C_2H_4-$ or

$$-\underset{\underset{CH_3}{|}}{CH}-CH_2-,$$

X is a hydrogen atom or a group of the general formula

$$R_1-\overset{\overset{O}{\|}}{C}-$$

n is a number from 1 to 5, preferably from 1 to 2,

B) from 2 to 15% by weight of a fatty acid alkanolamide of the general formula II

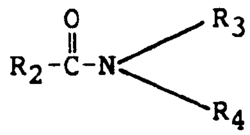

(II)

where

$R_2$ is a linear or branched, saturated or unsaturated hydrocarbon chain of from 9 to 23 carbon atoms,

$R_3$ and $R_4$ are independently of one another a hydrogen atom or a group of the formula $-C_2H_4OH$ or

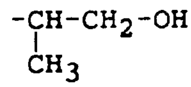

C) from 0.1 to 5% by weight of an amine of the general formula III

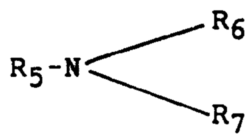

(III)

where

$R_5$ is a linear or branched, saturated or unsaturated hydrocarbon group of from 8 to 24 carbon atoms,

$R_6$ is a hydrogen atom or a linear or branched carbon chain of from 1 to 4 carbon atoms or a group of the formula $-(A-O)_m-H$, where A is as defined above,

$R_7$ is a group of the formula $-(A-O)_oH$ or a polyhydroxyalkyl radical of from 3 to 7, preferably from 4 to 6, carbon atoms which may be glycosidically linked to a mono-, di- or oligosaccharide, where m and o are independently of one another an integer from zero to 40, preferably from 1 to 30, the sum total of which, m plus o, can be an integer from 1 to 50, from 0.1 to 5% by weight of a carboxylic acid of the general formula IV

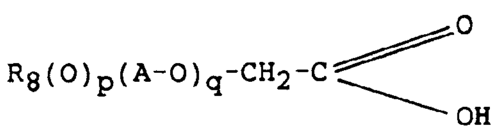

(IV)

where

$R_8$ is a linear or branched, saturated or unsaturated hydrocarbon group of from 7 to 22, preferably from 8 to 20, carbon atoms,

A is as defined in formula I,

p is O or 1, and

q is an integer from 0 to 16, p being equal to 1 when q is greater than 0,

E) from 0 to 5% by weight, preferably from 0.1 to 4% by weight, of a sulphonate of the general formula V

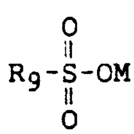

(V)

or of a sulphate of the general formula VI

$$R_{10}-O-(A-O)_n-\underset{\underset{\displaystyle O}{\|}}{\overset{\overset{\displaystyle O}{\|}}{S}}-OM \qquad (VI)$$

or of a mixture thereof, where

$R_9$ is a phenyl radical having from 1 to 3 substituents, preferably from the group of the hydrocarbon radicals, the total number of carbon atoms in these substituents being from 1 to 9, preferably from 1 to 6,

$R_{10}$ is a linear or branched hydrocarbon radical of from 1 to 10, preferably from 2 to 6, carbon atoms, M is one atom equivalent of a monovalent or divalent metal, preferably of an alkali metal, or of an ammonium or substituted ammonium group while

A and n are each as defined in formula I,

F) from 0 to 2% by weight, preferably from 0 to 1% by weight, of a monovalent or divalent metal salt, preferably an alkali metal sulphate or alkali metal chloride, and also

G) water to 100% by weight.

2. Use Of the free-flowing dispersion of pearly lustre according to claim 1 as addition to aqueous surfactant preparations.

## Revendications

1. Dispersions irisées fluides constituées :

A) par 5 à 25 % en poids d'un ester glycolique d'acide gras de la formule générale

$$R_1-\overset{\overset{\displaystyle O}{\|}}{C}-(O-A)_n-O-X \qquad (I),$$

dans laquelle

$R_1$ représente un groupe hydrocarboné linéaire ou ramifié, saturé ou non-saturé, comportant de 14 à 23 atomes de carbone,

A représente un groupe de formule - $C_2H_4$ - ou de formule

$$-\underset{\underset{\displaystyle CH_3}{|}}{CH}-CH_2- \quad ,$$

X représente un atome d'hydrogène ou un groupe de la formule générale

$$R_1-\overset{\overset{\displaystyle O}{\|}}{C}- \quad ,$$

<u>n</u> représente un nombre d'une valeur de 1 à 5, de préférence de 1 à 2,

B) par 2 à 15 % en poids d'un alkanolamide d'acide gras de la formule générale

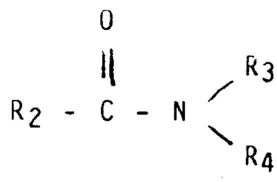

(II),

dans laquelle

$R_2$ représente un groupe hydrocarboné linéaire ou ramifié, saturé ou non-saturé, comportant de 9 à 23 atomes de carbone,

$R_3$ et $R_4$ indépendamment l'un de l'autre représentent un atome d'hydrogène ou un groupe de formule - $C_2H_4OH$, ou de formule

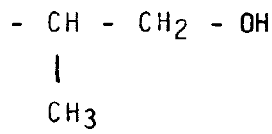

,

C) par 0,1 à 5 % en poids d'une amine de la formule générale

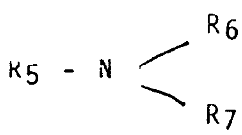

(III),

dans laquelle

$R_5$ représente un groupe hydrocarboné linéaire ou ramifié, saturé ou non-saturé, comportant de 8 à 24 atomes de carbone,

$R_6$ représente un atome d'hydrogène ou une chaîne carbonée linéaire ou ramifiée, ou un groupe de formule - $(A - O)_m$ - H, dans laquelle

A possède la signification indiquée ci-dessus,

$R_7$ représente un groupe de formule - $(A - O)_o$ - H, ou un radical poly-hydroxy-alkyle comportant de 3 à 7, de préférence de 4 à 6 atomes de carbone, qui est éventuellement relié de façon glycosidique avec un mono-, di- ou oligo-saccharide,

m et o représentant chacun, indépendamment l'un de l'autre, des nombres entiers d'une valeur de zéro à 40, de préférence de 1 à 30, dont la somme m + o peut être un nombre entier d'une valeur de 1 à 50,

D) par 0,1 à 5 % en poids d'un acide carboxylique de la formule générale

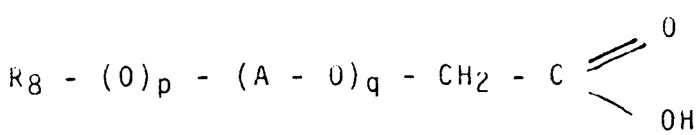

(IV),

dans laquelle

$R_8$ représente un groupe hydrocarboné linéaire ou ramifié, saturé ou non-saturé, comportant de 7 à 22, de préférence de 8 à 20 atomes de carbone,

A à la même signification que dans la formule (I),

p est égal à zéro ou à l'unité, et

q représente un nombre entier compris entre zéro et 16, p étant égal à l'unité lorsque q est supérieur à zéro,

E) par zéro à 5 % en poids, de préférence par 0,1 à 4 % en poids d'un sulfonate de la formule générale

$$R_9 - \overset{\displaystyle O}{\underset{\displaystyle O}{\overset{\|}{\underset{\|}{S}}}} - OM \qquad (V),$$

ou un sulfate de la formule générale

$$R_{10} - O - (A - O)_n - \overset{\displaystyle O}{\underset{\displaystyle O}{\overset{\|}{\underset{\|}{S}}}} - OM \qquad (VI),$$

ou un mélange de ceux-ci , dans lesquels

$R_9$ représente un radical phényle comportant de 1 à 3 substituants, de préférence dans le groupe des radicaux hydrocarbonés, la somme des atomes de carbone de ces substituants pouvant être un nombre de 1 à 9, de préférence de 1 à 6,

$R_{10}$ représente un reste hydrocarboné linéaire ou ramifié, comportant de 1 à 10 , de préférence de 2 à 6 atomes de carbone,

M est un atome-équivalent d'un métal monovalent ou bivalent, de préférence d'un métal alcalin, ou bien un groupe ammonium ou un groupe ammonium substitué, tandis que

A et n ont les significations indiquées dans la formule (I),

F) par zéro à 2 % en poids, de préférence par zéro à 1 % en poids, d'un sel métallique monovalent ou bivalent, de préférence d'un sulfate alcalin ou d'un chlorure alcalin, ainsi que

G) par de l'eau pour compléter à 100 % en poids.

2. L'utilisation de la dispersion irisée fluide selon la revendication 1 en tant qu'additif à des préparations aqueuses de surfactif.